(19) Europäisches Patentamt
European Patent Office
Office européen des brevets

(11) Veröffentlichungsnummer: **0 578 067 A1**

(12) # EUROPÄISCHE PATENTANMELDUNG

(21) Anmeldenummer: **93110109.1**

(22) Anmeldetag: **24.06.93**

(51) Int. Cl.5: **G01N 33/533**, C07D 405/08, C07D 405/10, C07D 405/12, C07D 213/38, C07H 21/00, C12Q 1/68

(30) Priorität: **07.07.92 DE 4222255**

(43) Veröffentlichungstag der Anmeldung: **12.01.94 Patentblatt 94/02**

(84) Benannte Vertragsstaaten: **CH DE FR GB IT LI NL SE**

(71) Anmelder: **BAYER AG**
**D-51368 Leverkusen(DE)**

(72) Erfinder: **Löbberding, Antonius, Dr.**
**Am Rohm 105**
**D-42113 Wuppertal(DE)**
Erfinder: **Mikhail, Gamal K.,Dr.**
**Küchenberger Strasse 33**
**D-51519 Odenthal(DE)**
Erfinder: **Springer, Wolfgang, Dr.**
**Katernberger Schulweg 31**
**D-42113(DE)**
Erfinder: **Hugl, Herbert, Dr.**
**Gemarkenweg 9**
**D-51467 Bergisch Gladbach(DE)**
Erfinder: **Köcher, Jürgen, Dr.**
**Seidenweberstrasse 5**
**D-40764 Langenfeld(DE)**

(54) **Fotochemische Markierung von Nukleinsäuren mit Europiumchelat-Reagenzien und ihre Verwendung in Gensondentestsystemen.**

(57) Die vorliegende Erfindung betrifft fotochemische Markierungsreagenzien enthaltend eine Lanthanidionen-chelatisierende Struktur und ein Furocumarin-Derivat verbunden über einen Spacer. Die Markierungsreagenzien werden in der Gensondendiagnostik eingesetzt.

EP 0 578 067 A1

Die Gensondendiagnostik ist eine Methode zum sequenzspezifischen Nachweis von DNA/RNA-Sequenzen. Sie basiert auf der Hybridisierung der Gensondensequenz mit komplementären Sequenzbereichen der nachzuweisenden DNA/RNA [J. A. Matthews, L. J. Kricka, Analytical Biochemistry 169, 1-25 (1988); U. Landegren, R. Kaiser, C.T. Caskey, L. Hood, Science 242, 229 (1988)].

Die Gensondendiagnostik ermöglicht den Nachweis von Infektionskrankheiten und genetischen Defekten. Voraussetzung für eine breite Anwendung der Gensondendiagnostik ist eine ausreichende Nachweisempfindlichkeit, eine einfache Durchführbarkeit sowie die Vermeidung von Radioaktivität.

Eine Variante der Gensondendiagnostik verläuft über die direkte fotochemische Markierung der nachzuweisenden DNA/RNA; anschließend erfolgt eine Hybridisierung an Gensonden mit komplementären Nukleinsäuresequenzen [N.Dattagupta, P. M. M. Rae, E. D. Huguenel, E. Carlson, A. Lyga, J. S. Shapiro, J. P. Albarella, Analytical Biochemistry 177, 85 (1989); J. P. Albarella, R. L. Minegar, W. L. Patterson, N. Dattagupta, E. Carlson, Nucleic Acids Research 17, 4293 (1989)].

Furocoumarine, die über geeignete Spacermoleküle mit Biotin verknüpft sind, erwiesen sich als gut geeignet zur Fotobiotinylierung von Nukleinsäuren. Nach der Hybridisierung an eine Gensonde mit einer komplementären Nukleinsäuresequenz und einem Trennschritt erfolgt die Detektion, beispielsweise durch Zufügen eines Komplexes aus Antibiotin-Antikörper bzw. Avidin oder Streptavidin mit alkalischer Phosphatase. Zum Nachweis wird in einem weiteren Schritt eine Farbreaktion durchgeführt, die durch alkalische Phosphatase ausgelöst wird [J. J. Leary, D. J. Brigati, D. C. Ward, Proc. Natl. Acad. Sci. USA 80, 4045-4049 (1983)].

Ein Nachteil des Nachweissystems über Biotin ist die weite Verbreitung von Biotin in biologischen Systemen.

Eine mögliche Alternative wäre die eine direkte Foto-Markierung der nachzuweisenden DNA/RNA beispielsweise mit einem Fluoreszenzfarbstoff. Dies erwies sich jedoch wegen bevorzugtem energy wasting unter den Bedingungen der Fotoreaktion als nicht durchführbar. Darüberhinaus müßte ein geeigneter Marker fotoinert sein.

Überraschenderweise erwiesen sich Lanthanidchelate, die über einen Spacer mit geeigneten Furocoumarinen verknüpft sind, als geeignet.

Lanthanidchelate, insbesondere Europiumchelate, werden bereits routinemäßig in der Immundiagnostik eingesetzt [P. Degan, A. Abbondandolo, G. Montangnoli, J. of Bioluminescence and Chemiluminescence 5, 207 (1990)]. Von besonderem Vorteil bei ihrer Anwendung ist die Möglichkeit der zeitaufgelösten Messung von Fluoreszenzlicht. Erste Anwendungen in der Gensondendiagnostik wurden inzwischen ebenfalls beschrieben [A. Oser, W. K. Roth, G. Valet, Nucleic Acids Res., 3, 1181 (1988)], die Markierung mit Europiumchelatreagenzien erfolgt hier jedoch in einem aufwendigen Verfahren. Weiterhin ist die Verwendung von Europiumchelatprimern für die PCR-Reaktion beschrieben [P. Dahlen, A. Iitiä, V.-N. Mukkala, P. Hurskainen, M. Kwiatkowski, Molecular and Cellular Probes 5, 143-149 (1991)].

Erfindungsgemäß wird ein Markierungsreagenz der allgemeinen Formel

Ln-S-Fu

synthetisiert, wobei:

- Ln = eine Lanthanidionen-chelatisierende Struktur,
- S = ein Spacer Molekül und
- Fu = ein Furocumarin-Derivat als fotochemisch verknüpfbare Struktur.

Die Lanthanidionen-chelatisierende Struktur (Ln) ist ein Pyridinderivat der Formel

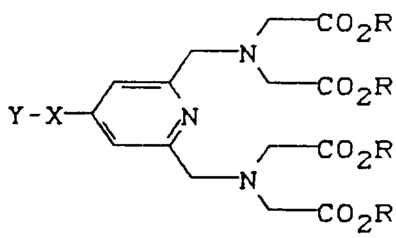

wobei

X für gegebenenfalls Heteroatomgruppierung enthaltendes $C_5$- bis $C_{14}$-Arylen oder $C_1$- bis $C_{24}$, Heteroatomgruppierungen enthaltendes [N, O, S (1x, mehrfach)] Alkylen steht,

Y und gegebenenfalls X + Y für N-Oxysuccinimido, N-Maleinsäureimido, $NH_2$, OH, $COCH_2$-Halogen, Halogen, NCO, NCS, CHO, COOH, SH, CO-Halogen, $COOCOR^1$, $CH=CHCO_2R^1{}_2$,

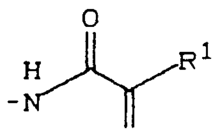 , 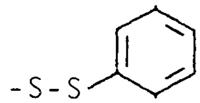 , 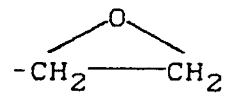 ,

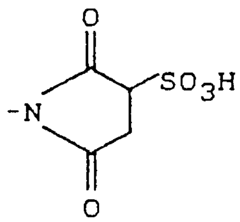 , 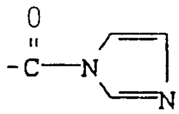

steht, wobei $R^1$ für Wasserstoff, einen gesättigten oder ungesättigten, gegebenenfalls durch eine Phenylgruppe substituierten $C_1$- bis $C_{20}$-Alkylrest oder eine Phenylgruppe steht,

R unabhängig voneinander jeweils für Wasserstoff, Ammonium oder ein Äquivalent eines Alkalimetalls oder 1/2 Äquivalent eines Erdalkalimetalls steht.

Die Synthese des Pyridin-Derivates Ln erfolgt nach an sich bekannten Methoden [s. z.B. F. Vögtle and C. Ohm, Chem. Ber. 117, 849 bis 854 (1984); R. Singh and G. Just, J. Org. Chem. 54, 4453 (1989)].

Der Spacer ist ein Polyalkylamin, Polyethylenglykol oder eine Kombination davon.

Polyalkylamine haben folgende allgemeine Formel:

$$-\overset{\displaystyle R}{\overset{|}{N}}-[-(CH_2)_x-\overset{\displaystyle R}{\overset{|}{N}}-]_y-$$

wobei

R für H, $C_1$-$C_7$-Alkyl, Aryl (wie z.B. Phenyl, Naphthyl oder Anthracyl), Hydroxy oder $C_1$-$C_7$-Alkoxy;

x für eine Zahl zwischen 2 und 7;

Y für eine Zahl zwischen 3 und 10 steht.

R kann in den unterschiedlich möglichen o.g. Varianten vorkommen, d.h. muß nicht identisch sein bei jeder Wiederholung der

$$(CH_2)_x-\overset{\displaystyle R}{\overset{|}{N}}\text{-Einheit}$$

im Spacer. Das gleiche gilt auch für x, d.h. x muß nicht identisch sein bei jeder Wiederholung der $-(CH_2)_x$-Einheit im Spacer.

Bevorzugt sind unabhängig voneinander die R's = H, $C_1$-$C_4$-Alkyl (z.B. Methyl, Ethyl, n-Propyl, i-Propyl, n-Butyl, i-Butyl, tert.-Butyl); x = 2, 3, 4 oder 5; und y = 3, 4, 5 oder 6.

Besonders bevorzugt sind N-4, N-9-Dimethylspermin-Derivate der Formel

$$-\overset{\displaystyle H}{N}-(CH_2)_3-\overset{\displaystyle CH_3}{\overset{|}{N}}-(CH_2)_4-\overset{\displaystyle CH_3}{\overset{|}{N}}-(CH_2)_3-\overset{\displaystyle H}{N}-$$

Polyethylenglykole haben folgende allgemeine Formel

$-O-[-(CH_2)_x-O-]_y-$

wobei

x = 2, 3, 4 oder 5 und

y = 3, 4, 5 oder 6 ist.

Bevorzugt sind Polyethylenglykole mit x = 2, 3, 4 oder 5; y = 3, 4, 5 oder 6. Besonders bevorzugt sind Polyethylenglykole mit x = 2 und y = 4, 5 oder 6.

Spacer Moleküle mit kombinierten Amin/Glykolstrukturen haben folgende allgemeine Formel:

$$-Z^1-[-(CH_2)_x-Z^2-]_{y-1}-(CH_2)_x-Z^3-$$

wobei

| | |
|---|---|
| $Z^1$, $Z^2$ und $Z^3$ | unabhängig voneinander für 0 oder NR stehen, |
| R | für H, $C_1$-$C_7$-Alkyl, Aryl (wie z.B. Phenyl, Naphthyl oder Anthracyl), Hydroxy oder $C_1$-$C_7$-Alkoxy; |
| x | für eine Zahl zwischen 2 und 7; |
| Y | für eine Zahl zwischen 3 und 10 steht. |

Bevorzugt sind Spacer-Strukture mit $Z^2$ = O und $Z^1$, $Z^2$ = NR wobei die R's = H, $C_1$-$C_4$-Alkyl (z.B. Methyl, Ethyl, n-Propyl, n-Butyl, i-Butyl, tert.-Butyl); x = 2, 3, 4 oder 5; und y = 3, 4, 5 oder 6.

Besonders bevorzugt sind Strukturen mit $Z^2$ = O,
$Z^1$, $Z^3$ = NR,
R = H, Methyl, Ethyl,
X = 2,
Y = 6.

Geeignete fotochemisch verknüpfbare Strukturen sind insbesondere Furocumarine, wie z.B. Angelicin (Isopsoralen) bzw. Psoralene sowie Derivate davon die mit Nukleinsäure fotochemisch reagieren.

Angelicin-Derivate haben folgende allgemeine Formel:

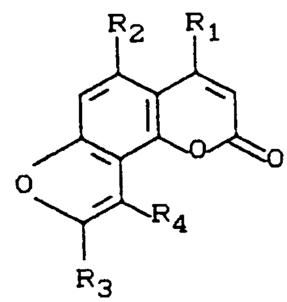

wobei
$R_1$, $R_2$ und $R_3$ unabhängig voneinander für H oder $C_1$-$C_7$-Alkyl, und $R_4$ für H, $C_1$-$C_7$-Alkyl oder niedrige Alkyl mit Hydroxy, $C_1$-$C_7$-Alkoxy-, Amino-, Halo- oder N-Phthalimidosubstituenten stehen.

Besonders bevorzugt sind Angelicin-Derivate die folgende $R_1$-$R_4$-Gruppierungen enthalten:

| $R^1$ | $R^2$ | $R^3$ | $R^4$ |
|---|---|---|---|
| H | H | H | H |
| $CH_3$ | H | $CH_3$ | H |
| $CH_3$ | $CH_3$ | $CH_3$ | $CH_2OH$ |
| $CH_3$ | H | $CH_3$ | $CH_2OCH_3$ |
| $CH_3$ | H | $CH_3$ | $CH_2NH_2$ |
| H | H | $CH_3$ | $CH_2Cl$ |
| H | H | $CH_3$ | 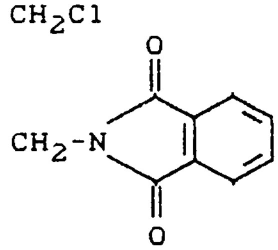 |

Andere Verbindungen mit unterschiedlichen R's können auch nach literaturbekannten Verfahren synthetisiert werden.

Geeignete Psoralene haben folgende allgemeine Formel:

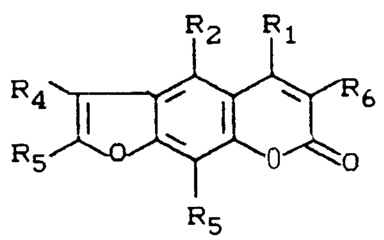

worin

| | |
|---|---|
| $R^1$, $R^3$ und $R^6$ | unabhängig voneinander für H oder $C_1$-$C_7$-Alkyl, |
| $R_4$ | für H, $C_1$-$C_7$-Alkyl oder $C_1$-$C_7$-Alkyl mit Hydroxy-, $C_1$-$C_7$-Alkoxy-, Amino-, Halo- oder N-Phthalimidosubstituenten, |
| $R_2$ und $R_5$ | unabhängig voneinander für H, Hydroxy, Carboxy, Carbo-$C_1$-$C_7$-alkoxy oder $C_1$-$C_7$-Alkoxy steht. |

Angelicin-Derivate sind vorteilhaft im Vergleich zu Psoralenen aufgrund der Monoadukt-Bildung.

Die Reihenfolge der Bindung des Lanthanidion-chelatbildners, des Spacers-, und des Furocumarins ist beliebig. Es ist also unter anderem möglich, zuerst den Chelatbildner Ln mit dem Spacer S zu verknüpfen und anschließend mit dem Furocumarin Fu umzusetzen. Umgekehrt kann Fu-S zuerst aufgebaut und dann mit Ln umgesetzt werden.

Die Verknüpfung der Bausteine erfolgt in an sich bekannter Weise.

## Beispiele

### Beispiel 1a)

Herstellung von 2,6-Bis[N,N-bis(t-butoxycarbonylmethyl)-amino-methyl]-4-(5-hydroxypent-1-inyl)pyridin (1):

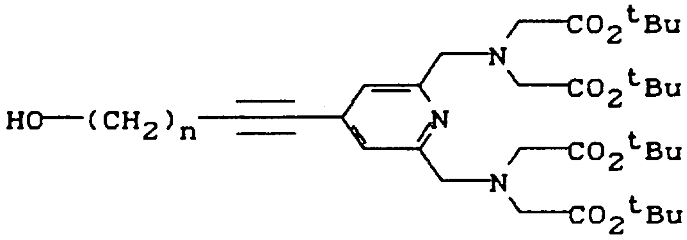

1 n = 3

2 = 9

6 g (10 mmol) 2,6-Bis[N,N-bis(t-Butoxycarbonylmethyl)-amino-methyl]-4-brompyridin (hergestellt wie von H. Tokalo, P. Pasanen und J. Kaukare in Acta Chem. Scand. Ser. B 42, (1988) 373 beschrieben) werden in einem Gemisch aus frisch destilliertem Tetrahydrofuran, 15 ml und 15 ml Triethylamin gelöst. Die Lösung wird entgast. 1 g (12 mmol) 5-Hydroxypent-1-in wird eingetragen. Der Katalysator bestehend aus einem Gemisch von 280 mg (0,4 mmol) Bis(triphenylphosphin)palladium(II)chlorid, 840 mg (3,2 mmol) Triphenylphosphin und 117 mg (0,61 mmol) Cu(I)Iodid wird bei Raumtemperatur und unter Rühren zugegeben. Die Reaktion ist nach 7 Stunden Rückfluß laut DC beendet. Nach dem Abkühlen auf Raumtemperatur und anschließendem Filtrieren wird die Lösung im Vakuum eingeengt und über Kieselgel chromatographiert (Laufmittel: Essigsäureethylester, Rf = 0,61).

Man erhält 4,6 g (68 % der Theorie) eines leicht gelblichen Feststoffs vom Schmelzpunkt 90 °C.

Beispiel 1b)

Herstellung von 2,6-Bis[N,N-bis(t-butoxycarbonylmethyl)-amino-methyl-4-(11-hydroxyundec-1-inyl)-pyridin (2):
6 g (10 mmol) 2,6-Bis[N,N-bis(t-butoxycarbonylmethyl)-amino-methyl]-4-brompyridin werden mit 1,93 g (12 mmol) 1-Undecin-10-ol unter Pd-Katalyse analog Beispiel 1a) umgesetzt. Man erhält nach Chromatographie auf Kieselgel (Laufmittel: Essigsäureethylester, Rf. = 0,52) 7 g (77 % der Theorie) einen gelben Feststoff vom Schmelzpunkt 57 bis 59 °C.

Beispiel 2a)

Herstellung von 2,6-Bis[N,N-bis(t-butoxycarbonylmethyl)-amino-methyl]-4-(5-hydroxypentanyl)pyridin (3):

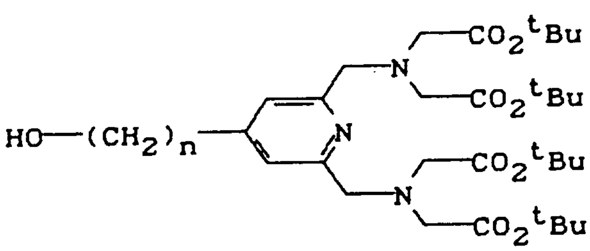

3 n = 5

4 = 11

472 mg (0,7 mmol) der in Beispiel 1 beschriebenen Verbindung 1 wird in 20 ml abs. Ethanol gelöst und mit 24 mg 10 %iger Pd/C versetzt. Unter positivem Druck von Wasserstoff wird die Lösung bei 45 bis 50 °C stark gerührt. Innerhalb 1 Stunde ist die Reaktion (lt. DC) beendet. Nach dem Abkühlen und Abtrennen des Katalysators wird die Losung im Vakuum eingeengt und der Rückstand wird auf Kieselgel chromatographiert (Laufmittel: Essigsäureethylester, RF = 0,52). Man erhält 242 mg (51 % der Theorie) eines leicht gelblichen Öls.

Eine Verbesserung der Ausbeute (56 % der Theorie) wird erzielt, wenn man $PtO_2$ als Katalysator unter den gleichen Reaktionsbedingungen verwendet.

Beispiel 2b)

Herstellung von 2,6-Bis[N,N-bis(t-butoxycarbonylmethyl)-amino-methyl-4-(11-hydroxyundecanyl)pyridin (4):

1,0 g (1,32 mmol) der in Beispiel 1b) beschriebenen Verbindung 2 wird unter $PtO_2$-Katalyse (100 mg) analog Beispiel 2a) hydriert. Man erhält nach Chromatographie auf Kieselgel (Laufmittel: Chloroform/Ethanol 15 : 1, Rf. = 0,4) 725 mg (72 % der Theorie) eines leicht gelblichen Öls.

Beispiel 3

Herstellung von Amino-PEG-angelicin (5)

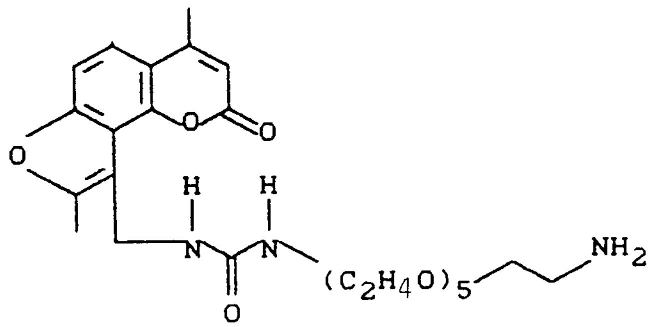

5

4,87 g (20 mmol) 4'-Aminomethyl-4,5'-dimethylangelicin werden in 25 ml DMF gelöst und bei Raumtemperatur mit 3,24 g (20 ml) Carbonyldiimidazol umgesetzt. Eine vollständige Umsetzung (lt. DC) wurde nach 6 Stunden Rühren unter Stickstoff festgestellt. Die Lösung wird zu einer Lösung aus 16,85 g (60 mmol), 1,17-Diamino-3,6,9,12,15-pentaoxaheptadecan in 40 ml DMF bei 80 °C langsam zugetropft und weiter bei 70 °C für 12 Stunden gerührt. Nach dem Abkühlen wird die Lösung im Vakuum eingeengt und auf Kieselgel chromatographiert (Laufmittel: Chloroform, Methanol, Ammoniak 90:10:1, Rf. = 0,28). Man erhält 7,1 g (65 % der Theorie) eines leicht gelben Öls.

Beispiel 4a)

Herstellung von Ln-S-Fn-Ester (6):

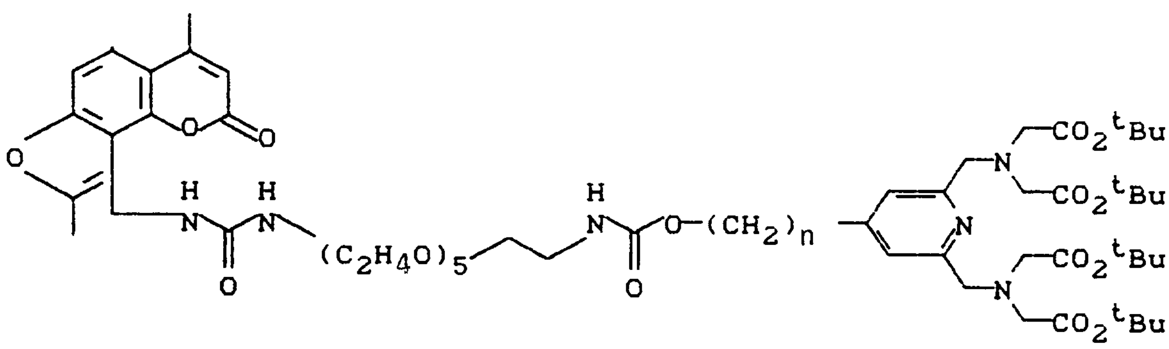

6 n = 5

7 = 11

250 mg (0,37 mmol) der in Beispiel 2a) beschriebenen Verbindung 3 werden in 3 ml trockenem Toluol gelöst. 65 mg (0,4 mmol) Carbonyldiimidazol werden zugegeben. Nach 17 Stunden Rühren bei 60 °C unter $N_2$ ist 2 total abreagiert (lt. DC, Laufmittel: Chloroform/Ethanol 15:1, Rf. = 0,45) und ein neues Produkt entstanden (Laufmittel: s.o., Rf. = 0,63). 220 mg (0,4 mmol) der in Beispiel 3 beschriebenen Verbindung 5 werden zugegeben und das Reaktionsgemisch bei 90 °C für weitere 24 Stunden gerührt. Nach dem Abkühlen wird die Lösung im Vakuum eingeengt und der Rückstand auf Kieselgel chromatographiert (Laufmittel: Toluol/Ethanol 5 : 1, Rf. = 0,36). Man erhält 138 mg (30 % der Theorie) eines leicht gelben Öls.

Beispiel 4b)

Herstellung von Ln-S-Fn-Ester (7):
410 mg (0,54 mmol) der in Beispiel 2b) beschriebenen Verbindung 4 werden mit Carbonyldiimidazol aktiviert und anschließend mit dem in Beispiel 3) beschriebenen Amino-PEG-angelicin 5 analog Beispiel 4a) umgesetzt. Man erhält nach der Chromatographie auf Kieselgel (Laufmittel: Toluol/Ethanol 5:1, Rf. = 0,31) 188 mg (26 % der Theorie) eines gelblichen Öls.

Beispiel 5a)

Herstellung von Ln-S-Fn-Tetracarbonsäure (8):

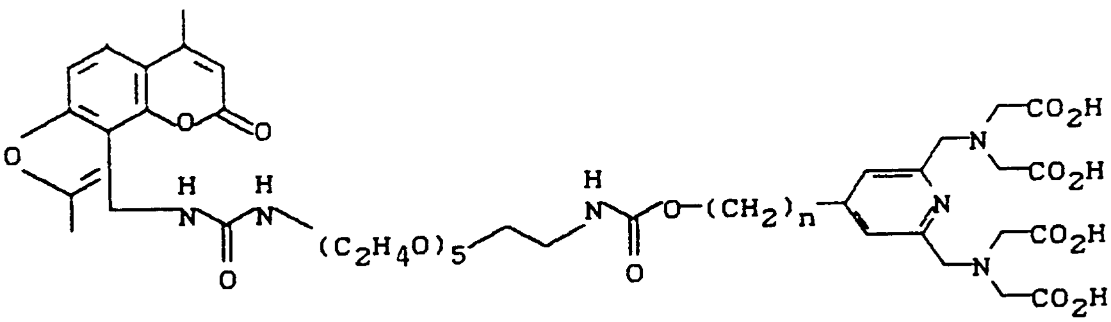

8 n = 5

9 = 11

138 mg (0,11 mmol) der in Beispiel 4a) beschriebenen Tetraester 6 werden in 4 ml trockenem Benzol gelöst und unter $N_2$ mit 569 g (5 mmol) Trifluoressigsäure versetzt. Nach 2 Stunden bei 60 °C Verrühren scheidet das Produkt in Benzol als Öl aus. Die Reaktion ist lt. DC beendet. Nach dem Abkühlen wird die Lösung im Vakuum eingeengt. Der Rückstand wird in 5 ml destilliertem Wasser gelöst und 2 x mit 3 ml Diethylether ausgeschüttelt. Die wäßrige Phase wird eingeengt und RP 18 chromatographiert (Laufmittel: Methanol, Rf. = 0,13). Man erhält 70 mg (62 % der Theorie) eines milchartigen, zähflüssigen Öls.

Beispiel 5b)

Herstellung von Ln-S-Fn Tetrasäure (9):
45 mg (0,034 mmol) der in Beispiel 4b) beschriebenen Tetraester 9 werden mit Trifluoressigsäure analog Beispiel 5a) umgesetzt. Man erhält 30 mg (81 % der Theorie) eines zähflüssigen Öls.

Beispiel 6

Herstellung von N1-(Angelicinamide)-N4,N9-dimethylspermin (10):

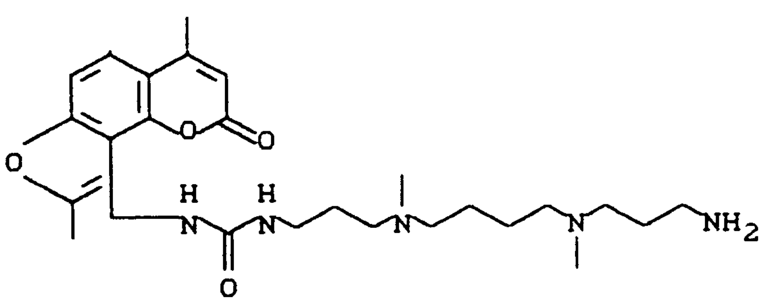

10

4,87 g (20 mmol) 4'-Aminomethyl-4,5'-dimethylangelicin werden mit Carbonyldiimidazol analog Beispiel 1 aktiviert. Die entstandene Lösung wird zu einer Lösung aus 13,8 g (60 mmol) N4,N9-Dimethylspermin in 40

ml DMF analog Beispiel 1 zugetropft. Nach dem Abkühlen wird die Lösung im Vakuum eingeengt und der Rückstand auf Kieselgel chromatographiert (Laufmittel: Chloroform/Methanol/Ammoniak 30:5:1, Rf. = 0,11). Man erhält 7,1 g (71 % der Theorie) eines gelben Öls.

Beispiel 7

Herstellung von Ln-S-Fn-Ester 11:

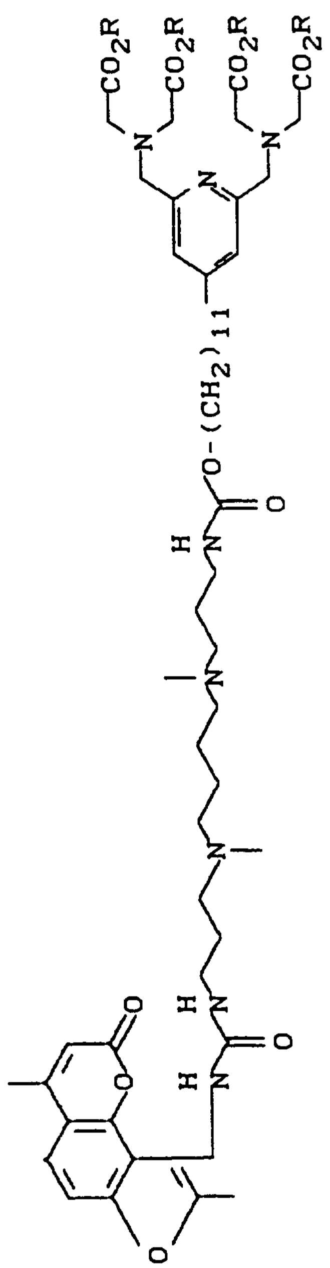

R = t-Butyl

318 mg (0,5 mmol) der in Beispiel 2b beschriebenen Verbindung 4 werden mit Carbonyldiimidazol aktiviert und anschließend mit der in Beispiel 6 beschriebenen Aminoverbindung 10 analog Beispiel 4 umgesetzt. Man erhält nach der Chromatographie auf Kieselgel (Laufmittel: Chloroform/Methanol/Ammoniak 70:45:1, Rf. = 0,42) 132 mg (21 % der Theorie) eines gelben Öls.

Beispiel 8

Herstellung von Ln-S-Fn-Tetrasäure 12: (EuPA)

30 mg (0,023 mmol) der in Beispiel 7 beschriebenen Tetraester 11 werden mit Trifluoressigsäure analog Beispiel 5 umgesetzt. Man erhält 22 mg (92 % der Theorie) eines gelben Öls.

Beispiel 9

Fotoreaktion von Hairpin-Oligonukleotiden mit EuPA (12)
50 µg des Hairpin-Oligonukleotids werden in 100 µl Tris-HCl-Puffer aufgenommen. Die Lösung wird im Wasserbad für 15 Minuten bei 50°C belassen. Zum langsamen Abkühlen auf Raumtemperatur wird die Probe aus dem Wasserbad genommen. Anschließend werden weitere 400 µl Wasser hinzugefügt.

Zur Fotoreaktion werden 15 µg des hybridisierten Hairpin-Oligonukleotids mit einem 20-fach molaren Überschuß an EuPA versetzt. Die Lösung wird im Anschluß unter einer UV-Lampe bei 312 nm oder 366 nm in einem Eppendorfgefäß in einem Eisbad belichtet. Die Fotoreaktion wird per HPLC verfolgt. Innerhalb von 15 Minuten war die Fotoreaktion abgeschlossen.

Beispiel 10

Fotomarkierung mit EuPA (12)
Zur Fotomarkierung mit EuPA wurden 2 bis 5 µg DNA in 20 µl TE-Puffer mit 50 µl 1M Natriumtetraboratpuffer pH 8,3 und 50 µl EuPA (2 µg/µl) versetzt und mit bidest. $H_2O$ auf 500 µl aufgefüllt. Der Ansatz wurde 10 Minuten bei 312 nm mit einem UV-Transilluminator bestrahlt, die Proben dabei auf Eis gelagert.

Die fotomarkierte DNA wurde anschließend mit 1/10 Volumen 3 M Natriumacetat pH 5,8 und 1 Volumen Isopropanol bei Raumtemperatur gefällt und 5 Minuten stehen gelassen. Anschließend wurde die DNA bei 10.000 UpM in einer Eppendorff-Zentrifuge abzentrifugiert, der Überstand abdekantiert und der DNA-Niederschlag mit 70 %igem Ethanol gewaschen. Nach der Trocknung der Proben wurde die fotomarkierte DNA in TE aufgenommen. Die Fotomarkierung der DNA mit EuPA wurde anschließend durch Agarosegelelektrophorese und Mikrotitertests überprüft.

Beispiel 11

Nachweis der EuPA-Markierung im Mikrotitertest
Zum Nachweis der EuPA-Markierung von doppelsträngiger DNA wurde die DNA nach der Markierung in Mikrotitertestplatten in Konzentrationen von 250 ng bis 125 pg in 1:2 Verdünnungsstufen pipettiert. Zur Anlagerung der DNA an die Polystyrolgruppen der Mikrotiter-Wells wurde die DNA zunächst in den Wells mit PBSM-Puffer (10 mM Naphosphat pH 7.2 mit 0,1 M $MgCl_2$, 0,15 M NaCl, 3 M KCl) verdünnt und über Nacht bei Raumtemperatur inkubiert. Anschließend wurde 2x mit 200 µl PBSM-Puffer gewaschen und die DNA durch 10-minütige Bestrahlung mit einem UV-Transilluminator bei 312 nm an den Wells fixiert. Die so fixierte DNA wurde anschließend mit Waschkonzentrat-Puffer der Firma Delfia/Pharmacia 4x gewaschen, um überschüssiges mit Europium beladenes EuPA zu entfernen. Als Negativkontrolle wurde nicht markierte, doppelsträngige DNA auf die gleiche Weise behandelt.

Nach Zugabe von 100 µl Enhancement-Lösung der Firma Wallac/-Pharmacia wurde nach 30 Minuten bei Raumtemperatur die zeitaufgelöste Fluoreszenz von Europium in einem Fluoreszenzfotometer DELFIA 1232 der Firma Wallac/Pharmacia bei 290 bis 360 nm Anregung/615 nm Emission gemessen. Bei der markierten DNA wurden je nach Verdünnung der DNA Fluoreszenzsignale von 212 000 bis 1 700 gemessen. Die nicht markierte DNA ergab nur geringe Backgroundsignale.

Beispiel 12

Hybridisierung mit EuPA markierter genomischer DNA im Reversed Phase Test
Die Herstellung von EuPA markierter DNA wurde nach der in Beispiel 10 beschriebenen Methode durchgeführt.

Die Hybridisierung wurde nach üblichen Verfahren durchgeführt bei einer Inkubationstemperatur von 40 bis 68°C. Je nach Hybridisierungstemperatur wurden unterschiedliche Substanzen zugesetzt. Dextransulfat oder andere Polymere wurden eingesetzt, um die Geschwindigkeit und das Ausmaß der Hybridisierung mit langen Genprobes zu erhöhen. Detergentien und Blockierungs-Reagentien wie Trockenmilch, Denhardt's Lösung, Heparin oder SDS wurden zugesetzt, um die unspezifische Bindung der DNA an die Membran zu unterdrücken. Denaturierende Agentien wie Harnstoff oder Formamid können eingesetzt werden, um die Schmelztemperatur der Hybride zu reduzieren, so daß niedrigere Hybridisierungstemperaturen angewandt werden können. Darüber hinaus können durch Zugabe von heterologer DNA die nicht spezifische Bindung von Probes an nicht homologer DNA auf dem Blot reduziert werden.

Zur Vorbereitung der Hybridisierung wurden 100 ng der nicht markierten E.coli spezifischen Genprobes/1,7 kb bis 6kb) zunächst 5 Minuten bei 100°C denaturiert, auf 0°C abgekühlt und dann auf

vorbehandelte Nitrozellulose oder Nylonmembranen mit Hilfe eines Minifold-II-Filtrationsgerätes der Firma Schleicher und Schüll aufgetragen und bei 80 °C 2 Stunden fixiert.

Die Filter wurden in einer versiegelten Plastik-Folientasche oder Plastikbox mit mindestens 20 ml Hybridisierungslösung pro 100 $cm^2$ Filter bei 68 °C mindestens 1 Stunde hybridisiert.

Die Lösung wurde durch 2,5 ml/100 $cm^2$ Filter Hybridisierungslösung ersetzt, der frisch denaturierte (5 Minuten, 100 °C) EuPA markierte genomische DNA von E.coli (1 µg) zugesetzt wurden. Die Filter wurden mindestens 6 Stunden bei 68 °C inkubiert unter schwachem Schütteln.

Die Filter wurden dann 2x 5 Minuten bei Zimmertemperatur mit mindestens 50 ml 2xSSC, 0,1 % SDS pro 100 $cm^2$ Filter und 2 x 15 Minuten bei 68 °C mit 0,1xSSC, 0,1 % SDS gewaschen.

Die Filter wurden dann direkt zur Detektion der hybridisierten DNA eingesetzt. Je nachdem, ob bereits mit Europium beladene EuPA-DNA verwendet wurde oder EuPA-DNA, die nachträglich mit Europium beladen wurde, ergaben sich folgende weitere Bearbeitungsschritte der Filter für den Fluoreszenz-Read Out. Bei nicht Europium beladener EuPA markierter genomischer DNA wurden die Filter in 100 µM EuCl, 100 µm EDTA und 1xSSC pH 7,0 in einem Gesamtvolumen von 2 ml 2 Stunden bei Raumtemperatur behandelt. Die Filter wurden dann sechsmal mit 2xSSC gewaschen. Anschließend wurden die einzelnen Slots des Hybridisierungsblots ausgeschnitten und in 1,5 ml Reaktionsgefäßen mit 1 ml Enhancement-Lösung behandelt. Nach 30-minütiger Inkubation bei Raumtemperatur wurden 200 µl von den Proben der einzelnen Slots in Mikrotiterplatten pipettiert und die Proben im Fluoreszenzfotometer DELFIA 1232 der Firma Wallac/Pharmacia bei 290 bis 360 nm Anregung und 615 Emission gemessen.

Bei Slot Blots mit EuPA markierter DNA, die vor der Markierung mit EuPA beladen wurde, wurden die einzelnen Slots direkt nach der Hybridisierung ausgeschnitten und in 1,5 ml Reaktionsgefäßen mit 1 ml Enhancement-Lösung versetzt und dann wie oben beschrieben, die Enhancement-Lösung zugegeben und im Fluoreszenzfotometer gemessen.

Lösungen:

| | |
|---|---|
| 20 x SSC: | 3M NaCl, 0,3 M Na-Citrat pH 7,0 |
| Hybridisierungslösung: | 5xSSC; 0,1 % N-Lauroylsarcosin, Na-Salz, 0,02 % SDS; 0,5 % Blocking Reagenz (Boehringer), Lösung bei 50 bis 70 °C lösen. |

## Beispiel 13

Hybridisierung mit EuPA markierten Genprobes

Die Herstellung von EuPA markierten Genprobes (1,7 bis 6 kb) wurde nach der in Beispiel 10 beschriebenen Methode durchgeführt.

Die EuPA markierten Genprobes können in Festphasen- oder Flüssighybridisierungen eingesetzt werden. Als Festphase eignen sich z.B. Nitrozellulose-, Nylonmembranen, Polystyrolgruppen von Mikrotiterplatten oder magnetische Partikel. Die fluoreszierenden Hybridisierungskomplexe von Genprobes mit komplementärer genomischer DNA können mit Hydroxylapatit von freien fluoreszierenden Genprobes abgetrennt werden.

Beispielhaft wurde eine Slot-Blot-Hybridisierung mit EuPA markierten E.coli spezifischen Genprobes (1,7kb bis 6kb) und genomischer DNA von E.coli durchgeführt.

Die genomische E.coli DNA wurde dazu 5 Minuten bei 100 °C denaturiert und dann auf 0 °C abgekühlt und dann auf Nitrozellulose oder Nylonmembranen mit Hilfe eines Minifold-II-Filtrationsgerätes der Firma Schleicher und Schüll in den Konzentrationen 500 ng bis 125 pg in 1:2 Verdünnungsschritten aufgetragen. Die Prähybridisierung und Hybridisierung wurden wie im Beispiel 12 beschrieben durchgeführt. 100 ng EuPA markierte E.coli Genprobe wurden eingesetzt.

Der Read Out erfolgte wie im Beispiel 12 beschrieben durch Einzelmessung der ausgeschnittenen Filter-Slots nach Behandlung mit Enhancement-Lösung in einem DELFIA Fluorometer 1232 der Firma Walac/Pharmacia.

Mit den Genprobes wurden 125 ng genomische DNA von E.coli noch gut nachgewiesen. Dies entspricht einer Testsensitivität von 0,1 pg DNA gemessen an der Hybridisierung von reiner pBR 322 Plasmidprobe gegen pBR322 DNA.

Alternativ wurden Mikrotiter-Hybridisierungstest durchgeführt. Die genomische E.coli DNA wurde dazu wie oben beschrieben denaturiert und dann von 10 ng bis 45 pg verdünnt in Mikrotiterwells pipettiert und über Nacht bei Raumtemperatur stehen gelassen. Anschließend wurde 2 x mit 200 µl PBSM-Puffer gewaschen und die DNA dann mit einem UV-Transilluminator 10 Minuten bei 312 nm fixiert. 200 µl Hybridisierungslösung (Beispiel 12) mit 10 ng EuPA markierter Genprobe wurden zugegeben und die

Hybridisierung mindestens 6 Stunden bei 68 °C inkubiert. Anschließend wurden die Mikrotiterwells 2x5 Minuten bei Zimmertemperatur mit 2 x 200 µl 2 x SSC, 0,1 SDS und 2 x 15 Minuten bei 50 °C mit 2 x 200 µl, 0,1 x SSC, 0,1 % SDS gewaschen.

Der Read Out erfolgte wie im Beispiel 12 nach Behandlung der Wells mit 100 µl Enhancementlösung in einem Delfia Fluorometer 1232 der Firma Wallac/Pharmacia.

**Patentansprüche**

**1.** Markierungsreagenz der allgemeinen Formel

Ln-S-Fu

wobei

Ln = eine Lanthanidionen-chelatisierende Struktur,
S = ein Spacer-Molekül und
Fu = ein Furocumarin-Derivat ist.

**2.** Markierungsreagenz nach Anspruch 1, wobei die Lanthanidionen-chelatisierende Struktur (Ln) ein Pyridinderivat der Formel

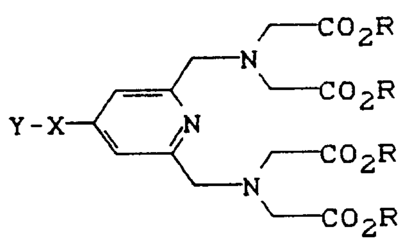

ist, wobei

X für gegebenenfalls Heteroatomgruppierungen enthaltendes $C_5$- bis $C_{14}$-Arylen oder $C_1$- bis $C_{24}$, Heteroatomgruppierungen enthaltendes [N, O, S (1x, mehrfach)] Alkylen steht,

Y und gegebenenfalls X + Y für N-Oxysuccinimido, N-Maleinsäureimido, $NH_2$, OH, $COCH_2$-Halogen, Halogen, NCO, NCS, CHO, COOH, SH, CO-Halogen, $COOCOR^1$, $CH=CHCO_2R^1$,

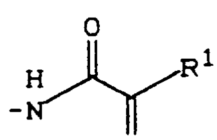 , 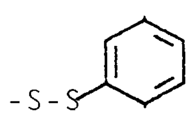 , 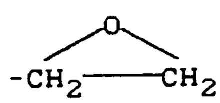 ,

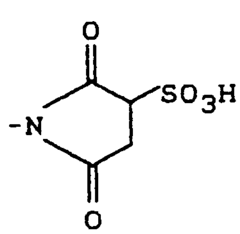 , 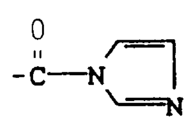

steht, wobei $R^1$ für Wasserstoff, einen gesättigten oder ungesättigten, gegebenenfalls durch eine Phenylgruppe substituierten $C_1$- bis $C_{20}$-Alkylrest oder eine Phenylgruppe steht,

R unabhängig voneinander jeweils für Wasserstoff, Ammonium oder ein Äquivalent eines Alkalimetalls oder 1/2 Äquivalent eines Erdalkalimetalls steht.

**3.** Markierungsreagenz nach Anspruch 1, wobei der Spacer ein Polyalkylamin, Polyethylenglykol oder eine Kombination davon ist.

4. Markierungsreagenzien nach Anspruch 1, worin Fu ein Angelicin-Derivat der folgende allgemeine Formel ist:

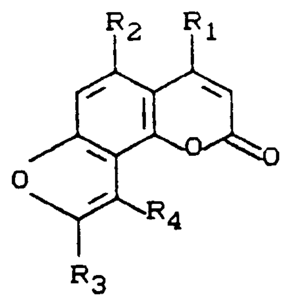

wobei
$R_1$, $R_2$ und $R_3$ unabhängig voneinander für H oder $C_1$-$C_7$- Alkyl, und $R_4$ für H, $C_1$-$C_7$-Alkyl oder niedrige Alkyl mit Hydroxy, $C_1$-$C_7$-Alkoxy-, Amino-, Halo- oder N- Phthalimidosubstituenten steht.

5. Markierungsreagenz nach Anspruch 1, worin Fu ein Psoralen mit folgender allgemeinen Formel ist:

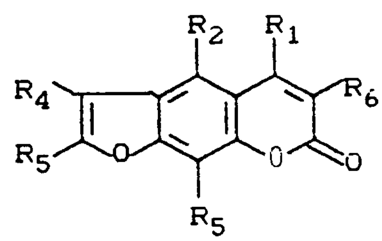

worin

| | |
|---|---|
| $R^1$, $R^3$ und $R^6$ | unabhängig voneinander für H oder $C_1$-$C_7$-Alkyl, |
| $R_4$ | für H, $C_1$-$C_7$-Alkyl oder $C_1$-$C_7$-Alkyl mit Hydroxy-, $C_1$-$C_7$-Alkoxy-, Amino-, Halo- oder N-Phthalimidosubstituenten, |
| $R_2$ und $R_5$ | unabhängig voneinander für H, Hydroxy, Carboxy, Carbo-$C_1$-$C_7$-alkoxy oder $C_1$-$C_7$- Alkoxy steht. |

Europäisches Patentamt

# EUROPÄISCHER RECHERCHENBERICHT

Nummer der Anmeldung

EP 93 11 0109
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | EP-A-0 203 047 (TAKALO,HARRY; PASANEN,PAAVO)<br>26. November 1986<br>* das ganze Dokument *<br>--- | 1-5 | G01N33/533<br>C07D405/08<br>C07D405/10<br>C07D405/12<br>C07D213/38<br>C07H21/00<br>C12Q1/68 |
| Y | ANNALS OF THE NEW YORK ACADEMY OF SCIENCES<br>Bd. 346, 1980, NEW YORK NY US<br>Seiten 355 - 367<br>PILL-SOON SONG AND CHING-NAN OU 'Labeling of Nucleic Acids with Psoralens'<br>* das ganze Dokument *<br>--- | 1-5 | |
| Y | EP-A-0 187 332 (MOLECULAR DIAGNOSTICS, INC.)<br>16. Juli 1986<br>* das ganze Dokument *<br>--- | 1-5 | |
| Y | NUCLEIC ACIDS RESEARCH<br>Bd. 16, Nr. 3, 11. Februar 1988, ARLINGTON, VIRGINIA US<br>Seiten 1181 - 1196<br>ANDREAS OSER, WILLY K. ROTH AND GÜNTER VALET 'Sensitive non-radioactive dot-blot hybridization using DNA probes labelled with chelate group substituted psoralen and quantitative detection by europium fluorescence'<br>* das ganze Dokument *<br>--- | 1-5 | RECHERCHIERTE SACHGEBIETE (Int. Cl.5 )<br>G01N<br>C12Q<br>C07H<br>C07D |
| D,A | MOLECULAR AND CELLULAR PROBES<br>Bd. 5, 1991, LONDON GB<br>Seiten 143 - 149<br>PATRIK DAHLEN ET AL. 'The use of europium (Eu3+) labelled primers in PCR amplification of specific target DNA'<br>* das ganze Dokument *<br>---<br>-/-- | 1-5 | |

Der vorliegende Recherchenbericht wurde für alle Patentansprüche erstellt

| Recherchenort | Abschlußdatum der Recherche | Prüfer |
|---|---|---|
| DEN HAAG | 30 SEPTEMBER 1993 | DOEPFER K.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 11 0109
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | THE JOURNAL OF BIOLUMINESCENCE AND CHEMILUMINESCENCE<br>Bd. 5, Nr. 3, Juli 1990, CHICHESTER GB<br>Seiten 207 - 212<br>PAOLO DEGAN, ANGELO ABBONDANDOLO AND GIORGIO MONTAGNOLI 'A New Fluorescence Enhancement Solution for Europium-based Time-resolved Fluoroimmunoassays'<br>* das ganze Dokument *<br>----- | 1-5 | |
| | | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| DEN HAAG | 30 SEPTEMBER 1993 | DOEPFER K.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)